# EUROPÄISCHE PATENTANMELDUNG

(11) **EP 3 239 193 A1**
(43) Veröffentlichungstag der Anmeldung: **01.11.2017**
(21) Anmeldenummer: 17166043.4
(22) Anmeldetag: 11.04.2017
(51) Int. Cl.: C08F 26/06, A61K 31/785, C02F 1/54, C08F 22/02, C08F 126/06, C08F 126/08, C08F 226/06, C08G 73/00, C08G 75/23

(54) **WASSERUNLÖSLICHE ANIONENAUSTAUSCHERMATERIALIEN**

(30) Priorität: 27.04.2016 DE 102016207128
(71) Anmelder: Leibniz-Institut für Polymerforschung Dresden e.V., 01069 Dresden (DE)
(72) Erfinder: MEIER-HAACK, Jochen, 01259 Dresden (DE)
(74) Vertreter: Rauschenbach, Marion

(57) **Zusammenfassung**

Die vorliegende Erfindung bezieht sich auf das Gebiet der Polymerchemie und betrifft wasserunlösliche Anionenaustauschermaterialien, wie sie beispielsweise für Anionenaustauschermembranen oder als Anionenaustauscherharze zum Einsatz kommen können.

Die Aufgabe der Erfindung besteht in der Angabe von wasserunlöslichen Anionenaustauschermaterialien, die eine verbesserte Wasserunlöslichkeit aufweisen.

Gelöst wird die Aufgabe durch wasserunlösliche Anionenaustauschermaterialien, mindestens bestehend aus miteinander linear polymerisierten und/oder verzweigten und/oder vernetzten Anionenaustauschergruppen C, die Bestandteil der Struktureinheiten gemäß mindestens einer der allgemeinen Formeln I bis VIII 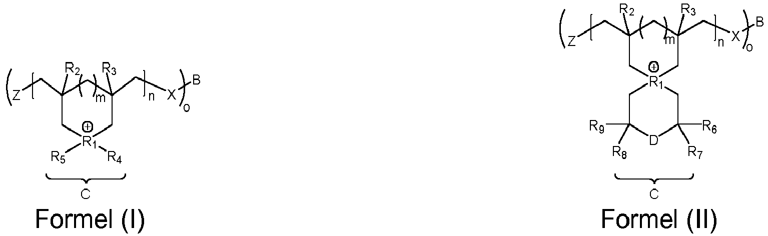 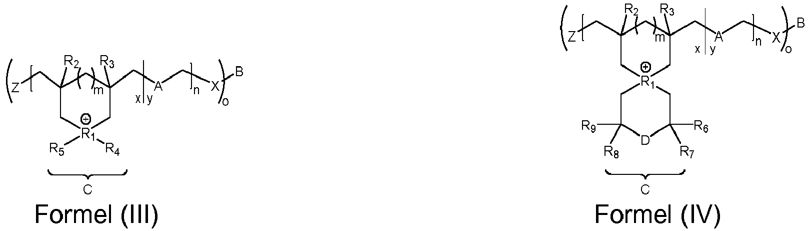 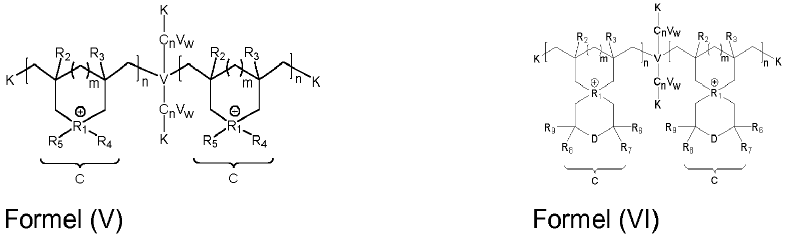 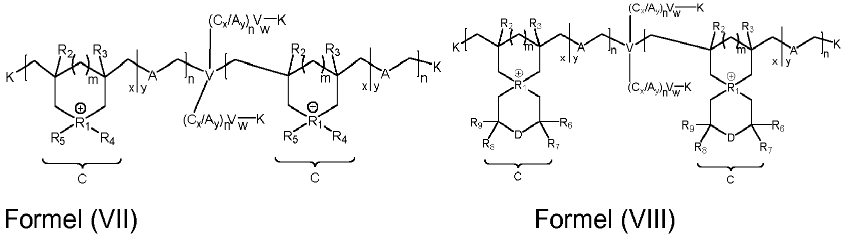 sind.

## Beschreibung

Die vorliegende Erfindung bezieht sich auf das Gebiet der Polymerchemie und betrifft wasserunlösliche Anionenaustauschermaterialien, wie sie beispielsweise für Anionenaustauschermembranen oder als Anionenaustauscherharze zur Wasseraufbereitung, in Katalysatoren, für elektrochemische Prozesse, wie Elektrodialyse oder Elektrodeionisation, oder in Energiewandlersystemen, wie Brennstoffzellen und Reverse-Elektrodialyse, oder Energiespeichersystemen, wie Redox-Flow-Batterien, zum Einsatz kommen können.

Es sind zahlreiche Anionenaustauschermaterialien bekannt.

Beispielsweise bestehen bekannte Anionenaustauscherharze aus vernetztem Polystyrol, die eine Benzyltrimethylammoniumgruppe als funktionelle Gruppe aufweisen. Für den Einsatz derartiger Anionenaustauscherharze in der OH-Form werden Betriebstemperaturen zwischen 35°C und maximal 60°C angegeben (Datenblatt Amberjet 9000 OH; Datenblatt DOWEX Marathon A; Datenblatt DOWEC Marathon 2; Dow Chemical Company).

Die Temperaturstabilität unterschiedlicher niedermolekularer quaternärer Ammoniumsalze in 6N NaOH bei 160°C wurde von Marino und Kreuer (ChemSusChem 8, 2015, 513-523) untersucht. Dabei wurde herausgefunden, dass die spirocyclische Verbindung Azonia-spiro[5.5]undecan unter den Testbedingungen die höchste Stabilität aufweist.

Aromatische Anionenaustauschermembranen auf der Basis von Polyethersulfonen mit N-spirocyclischen Ammoniumgruppen sind durch Pham und Jannasch, ACS Macro Letters 4 (2015) 1370-1375 bekannt. Die spirocyclischen Anionenaustauschergruppen sind dabei an einen Phenylring kondensiert und enthalten benzylische Methylengruppen.
Stabilitätsuntersuchungen an diesen Anionenaustauschermembranen in 1M Natronlauge haben einen teilweisen Abbau nach 168 h bereits bei 40°C gezeigt.

Gemäß Hellwinkel und Seifert weist Bis-2,2'-Biphenylenammoniumiodid eine hohe Thermostabilität in einem alkalischen Medium, wie 20-%ige Natronlauge, bei Siedetemperatur auf. Die Synthese dieser Verbindung ist jedoch sehr aufwändig und ihre Verwendung als Material für Anionenaustauschermembranen ist daher wirtschaftlich nicht sinnvoll (Liebigs Ann. Chem. 762 (1972) 29-54).

Ein Copolymer aus Chlortrifluorethylen und Diallyldimethylammoniumchlorid wird von Valade et al. als Bindermaterial für alkalische Brennstoffzellen angegeben (J. Polym. Sci.: Part A: Polym. Chem. 47, 2009, 2043-2205).
Nachteilig an dieser Verbindung ist, dass sie aufgrund ihrer geringen lonenaustauscherkapazität nicht als Membranmaterial eingesetzt werden kann.

Eine Anionenaustauschermembran auf der Basis eines Blends aus Poly(diallyldimethylammoniumchlorid) und Polyvinylalkohol ist durch Qiao et al. bekannt (J. Power Sources 237, 2013, 1-4). Zur Verringerung der Wasseraufnahme wurde das Material mit Glutaraldehyd vernetzt. Die lonenaustauscherkapazität der Membran blieb nach einem anfänglichen Verlust von 55 % der Kapazität innerhalb von 375 Stunden in 8N Kalilauge dann stabil.

Die Polymerisation und die Struktur von N,N-Diallylpyrrolidiniumbromid sind durch de Vynck et al. bekannt (Macromol. Rapid Commun. 18, 1997, 149-156).

Ein Nachteil der bekannten Lösungen für Anionenaustauschermaterialien ist, dass diese bei Temperaturen über 50 °C, insbesondere in einer alkalischen Umgebung, und hinsichtlich ihrer Wasserunlöslichkeit noch nicht ausreichend stabil sind.

Die Aufgabe der Erfindung besteht in der Angabe von wasserunlöslichen Anionenaustauschermaterialien, die eine verbesserte Wasserunlöslichkeit bei gleichzeitig verbesserter Alkali- und Temperaturstabilität, insbesondere bei Temperaturen über 50 °C, aufweisen.

Die Aufgabe wird durch die in den Ansprüchen angegebene Erfindung gelöst. Vorteilhafte Ausgestaltungen sind Gegenstand der Unteransprüche.

Die wasserunlöslichen Anionenaustauschermaterialien bestehen mindestens aus miteinander linear polymerisierten und/oder verzweigten und/oder vernetzten Anionenaustauschergruppen C, die Bestandteil der Struktureinheiten gemäß mindestens einer der allgemeinen Formeln I bis VIII 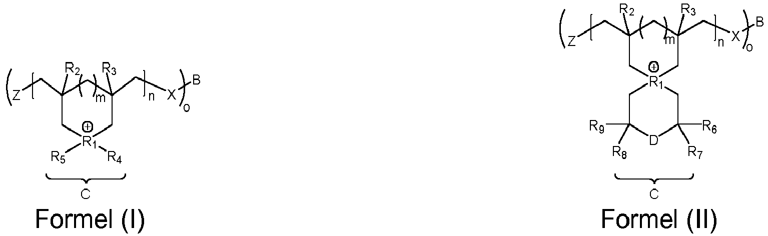 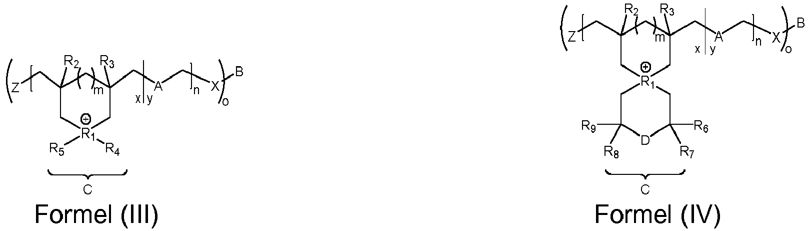 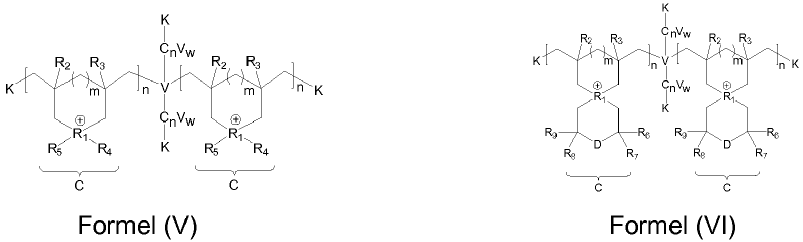 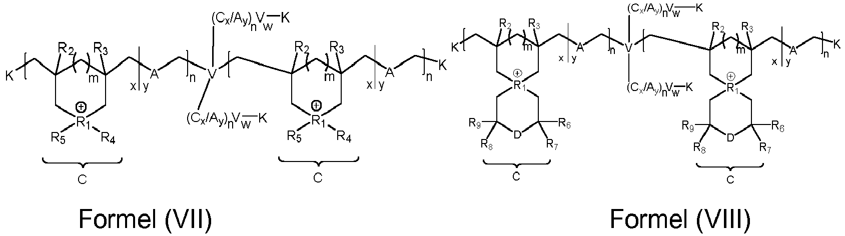 sind, mit
- R₁ Stickstoff und/oder Phosphor
- R₂ - R₉ Substituenten, wobei
   ∘ R₂ und R₃ Wasserstoff sind oder, im Falle dass mindestens ein Rest R₂ und R₃ ungleich Wasserstoff ist, dann R₂ und/oder R₃ ein Alkylrest oder Arylrest ist, und,
   ∘ R₄ und R₅ jeweils ein Alkyl- und/oder Arylrest ist, und
   ∘ R₆ - R₉ Wasserstoff sind, oder im Falle dass mindestens ein Rest R₆ bis R₉ ungleich Wasserstoff ist, dann diese Reste R₆ bis R₉ ein Alkylrest oder Arylrest ist, und
- Z eine Endgruppe ist, und
- X eine Verbindungsstelle ist, und
- V eine Vernetzungsstelle ist, und
- K eine Endgruppe Z oder eine Verbindungsstelle X ist, und
- D nichts ist oder mindestens eine Methylengruppe oder Sauerstoff oder Schwefel ist, und
- B ein wasserunlösliches temperaturstabiles und alkalistabiles Polymer ist, und
- A ein Comonomer ist, das keine funktionelle Gruppe aufweist, und
- 2 ≤ n ≤ 100 ist, und
- m = 0 oder 1 ist, und
- 1 ≤ o ≤ 100 ist, und
- 50 mol% ≤ x ≤ 99 mol% und y= (100 mol% -x) ist, und
- 10 mol% ≤ w ≤ 100 mol% und (n + w) = 100mol% ist,
wobei
- im Falle von K mindestens eine Endgruppe Z und mindestens eine Verbindungsstelle X vorhanden sind, und
- die Anionenaustauschergruppen C als Bestandteil der Struktureinheiten gemäß den allgemeinen Formeln I bis IV über eine, mehrere oder alle Verbindungsstellen X an ein wasserunlösliches Polymer B gekoppelt sind und im Wesentlichen alle Endgruppen Z eine temperatur- und alkalistabile Verbindung sind,
   und/oder
- die Anionenaustauschergruppe C als Bestandteil der Struktureinheiten gemäß den allgemeinen Formeln V bis VIII über die Vernetzungsstelle V miteinander vernetzt sind, und im Wesentlichen alle Endgruppen Z eine temperatur- und alkalistabile Verbindung sind und alle K, die eine Verbindungsstelle X sind, mit einem wasserunlöslichen Polymer B über kovalente chemische Bindungen gekoppelt sind,
   und/oder
- die Anionenaustauschergruppe C und das wasserunlösliche Polymer B über eine oder mehrere ionische Bindungen verbunden sind.

Vorteilhafterweise sind mindestens zwei Anionaustauschergruppen C gemäß der Formel I-IV mit einem wasserunlöslichen Polymer B über eine Verbindungsstelle X über kovalente chemische Bindungen miteinander gekoppelt und/oder die Anionenaustauschergruppen C sind über ionische Bindungen mit dem wasserunlöslichen Polymer B verbunden und die Endgruppen Z sind temperaturstabile und alkalistabile Verbindungen.

Weiterhin vorteilhafterweise ist die Verbindungsstelle X N,N-Diallylpiperidiniumether oder N,N-Diallylpyrrolidiniumether oder N,N-Diallylaminoethylether oder Methacrylamidethylether oder Methacrylsäureester oder Methacrylsäurehydroxypropylether oder Xylylenether oder Phenylenethersulfon.

Ebenfalls vorteilhafterweise sind Anionenaustauschergruppen C gemäß den Formeln V bis VIII über Vernetzungsstellen V vernetzt und K ist eine Verbindungsstelle X, an die das wasserunlösliche Polymer B über kovalente chemische Bindungen gekoppelt ist.

Eine weitere vorteilhafte Ausführung ist, dass das wasserunlösliche Polymer B über ionische Bindungen mit den Anionenaustauschergruppen C gemäß den allgemeinen Formeln I bis VIII verbunden ist.

Und auch vorteilhafterweise sind die Endgruppen Z Alkylthioether oder Arylthioether oder Benzylthioether.

Vorteilhaft ist es auch, wenn das wasserunlösliche, temperaturstabile und alkalistabile Polymer B Polyethersulfon oder Polythioethersulfon oder Polysulfon oder Polyphenylen oder Polyphenylenether oder Polyphenylensulfid oder Poly(perfluorethylenpropylen) oder Polytetrafluorethylen Poly(ethylentetrafluorethylen) oder Perfluoralkoxypolymere oder Polystyrol oder Polyethylen oder Polypropylen oder ein sulfoniertes und/oder carboxyliertes und/oder phosphoniertes Polymer des Typs Polyethersulfon oder Polysulfon oder Polyphenylen oder Polyphenylenether oder Polyphenylensulfid oder Poly(perfluorethylenpropylen) oder Polytetrafluorethylen Poly(ethylen-tetrafluorethylen) oder Perfluoralkoxypolymere oder Polystyrol oder Polyethylen oder Polypropylen ist.

Auch vorteilhaft ist es, wenn das Comonomer A Styrol und/oder α-Methylstyrol und/oder N-Vinylcarbazol und/oder Methacrylester und/oder N-Vinylpyrrolidon und/oder N,N-Diallylacrylamid und/oder N,N-Diallylacrylsulfonamid und/oder Diallylether und/oder 1,2-Diallylbenzol und/oder Diallylsulfid und/oder Chlortrifluorethylen und/oder Tetrafluorethylen und/oder Hexafluorpropylen und/oder 1,2-Divinylbenzol ist.

Ebenfalls vorteilhaft ist es, wenn die Vernetzungsstelle V Tetraallylammoniumchlorid oder Tetraalkylallylammoniumchlorid oder Diallyl-Dialkylallylammoniumchlorid oder 1,4-Divinylbenzol oder Divinylsulfon oder Divinylsulfid oder Divinylsulfoxid oder Divinylether oder Diacrylamid oder Dimethacrylamid oder N,N,N',N'-Tetraallyl-4,4'- Trimethylendipiperidininiumchlorid oder N,N,N',N'-Tetraalkylallyl-4,4'- Trimethylendipiperidiniumchlorid oder N,N-Diallyl-,N',N'Dialkylallyl-4,4'- Trimethylendipiperidiniumchlorid oder N-Allyl-N,N',N'Trialkylallyl-4,4'- Trimethylendipiperidiniumchlorid oder N,N,N'-Triallyl-N'-Alkylallyl-4,4'- Trimethylendipiperidiniumchlorid oder N,N,N',N'-Tetraallylpiperaziniumchlorid oder N,N,N',N'-Tetraalkylallylpiperaziniumchlorid oder N,N-Diallyl-N',N'Dialkylallylpiperaziniumchlorid oder N-Allyl-N,N',N'Trialkylallylpiperaziniumchlorid oder N,N,N'-Triallyl-N'-Alkylallylpiperaziniumchlorid und/oder ein Bromid und/oder ein Jodid dieser Verbindungen ist.

Weiterhin vorteilhaft ist es, wenn als Monomere für die Herstellung der Anionenaustauschergruppen C N,N-Diallyl- und/oder N,N-Dialkylallylverbindungen und/oder N-Allyl-N-Alkylallyl-Verbindungen sekundärer aliphatischer oder aromatischer oder cycloaliphatischer Amine, wie beispielsweise Diallyldimethylammoniumchlorid, Diallylpiperidiniumchlorid, Diallylpyrrolidiniumchlorid, Allyl-Methallyldimethylammoniumchlorid, Allyl-Methallylpiperidiniumchlorid, Allyl-Methallylpyrrolidiniumchlorid, Dimethallyldimethylammoniumchlorid, Dimethallylpiperidiniumchlorid, Dimethyallylpyrrolidiniumchlorid, Diallyl-3,4-Dimethylpyrrolidiniumchlorid, Diallyl-3,3,4,4-Tetramethylpyrrolidiniumchlorid, Diallyl-3,5-Dimethylpiperidiniumchlorid, Diallyl-3,3,5,5-Tetramethylpiperidiniumchlorid, Diallyl-Diphenylammoniumchlorid P,P-Diallyl- und/oder P,P-Dialkylallylverbindungen und/oder P-Allyl-P-Alkylallyl-Verbindungen sekundärer aliphatischer oder aromatischer oder cycloaliphatischer Phosphine, wie beispielsweise Diallyldimethylphophiumchlorid, Diallyldiphenylphosphoniumchlorid und/oder ein Bromid und/oder ein Jodid dieser Verbindungen eingesetzt sind.

Von Vorteil ist es auch, wenn R₁ Stickstoff ist.

Auch von Vorteil ist es, wenn R₂ und/oder R₃ ein Methylrest und/oder Wasserstoff ist.

Ebenfalls von Vorteil ist es, wenn R₄ und R₅ ein Alkylrest ist und vorteilhaft ein Methyl- oder Ethylrest ist.

Weiterhin von Vorteil ist es, wenn R₆ - R₉ Wasserstoff sind.

Und auch von Vorteil ist es, wenn 10 ≤ n ≤ 50 ist.

Vorteilhaft ist es auch, wenn 2 ≤ o ≤ 10 ist.

Mit der vorliegenden Erfindung ist es erstmals möglich, wasserunlösliche Materialien für Anionenaustauschermembranen anzugeben, die eine verbesserte Alkali- und Temperaturstabilität, insbesondere bei Temperaturen über 50 °C, aufweisen.

Erreicht wird dies durch wasserunlösliche Anionenaustauschermaterialien, mindestens bestehend aus miteinander linear polymerisierten und/oder verzweigten und/oder vernetzten Anionenaustauschergruppen C, die Bestandteil der Struktureinheiten gemäß mindestens einer der allgemeinen Formeln I bis VIII 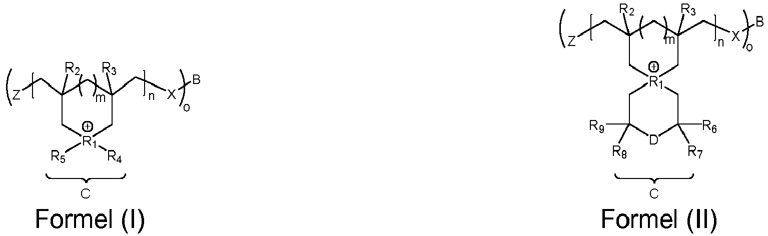 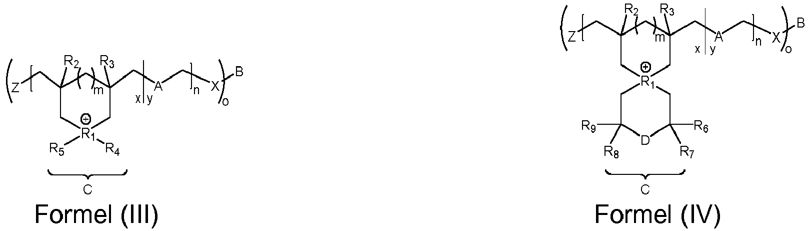 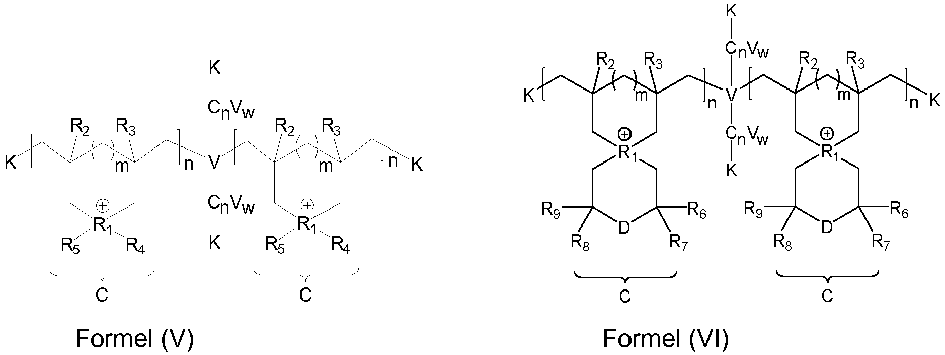 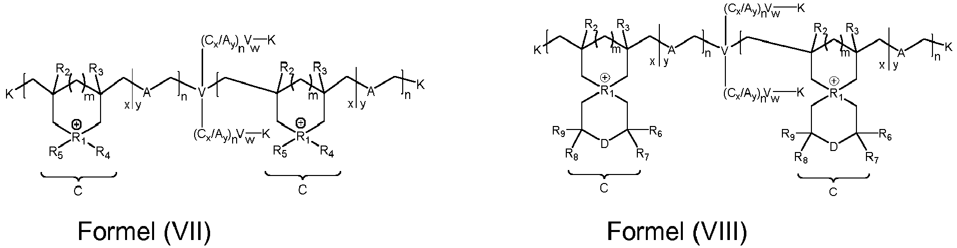 sind, mit
- R₁ Stickstoff und/oder Phosphor
- R₂ - R₉ Substituenten, wobei
   - R₂ und R₃ Wasserstoff sind oder im Falle, dass mindestens ein Rest R₂ und R₃ ungleich Wasserstoff ist, dann R₂ und/oder R₃ ein Alkylrest oder Arylrest ist, und,
   - R₄ und R₅ jeweils ein Alkyl- und/oder Arylrest ist, und
   - R₆ - R₉ Wasserstoff sind oder im Falle, dass mindestens ein Rest R₆ bis R₉ ungleich Wasserstoff ist, dann diese R₆ bis R₉ ein Alkylrest oder Arylrest ist,
   und
- Z eine Endgruppe ist, und
- X eine Verbindungsstelle ist, und
- V eine Vernetzungsstelle ist, und
- K eine Endgruppe Z oder eine Verbindungsstelle X ist, und
- D nichts ist oder mindestens eine Methylengruppe oder Sauerstoff oder Schwefel ist, und
- B ein wasserunlösliches Polymer ist, und
- A ein Comonomer ist, das keine funktionelle Gruppe aufweist, und
- 2 ≤ n ≤ 100 ist, und
- m = 0 oder 1 ist, und
- 1 ≤ o ≤ 100 ist, und
- 50 mol% ≤ x ≤ 99 mol% und y= (100 mol% -x) ist, und
- 10 mol% ≤ w ≤ 100 mol% und (n + w) = 100mol% ist..

Erfindungswesentlich ist, dass
- im Falle von K mindestens eine Endgruppe Z und mindestens eine Verbindungsstelle X vorhanden sind, und
- die Anionenaustauschergruppen C als Bestandteil der Struktureinheiten gemäß den allgemeinen Formeln I bis IV über eine, mehrere oder alle Verbindungsstellen X an ein wasserunlösliches Polymer B gekoppelt sind und im Wesentlichen alle Endgruppen Z eine temperatur- und alkalistabile Verbindung sind,
   und/oder
- die Anionenaustauschergruppe C als Bestandteil der Struktureinheiten gemäß den allgemeinen Formeln V bis VIII über die Vernetzungsstelle V miteinander vernetzt sind, und im Wesentlichen alle Endgruppen Z eine temperatur- und alkalistabile Verbindung sind und alle K, die eine Verbindungsstelle X sind, mit einem wasserunlöslichen Polymer B über kovalente chemische Bindungen gekoppelt sind,
   und/oder
- die Anionenaustauschergruppen C und das wasserunlösliche Polymer B über eine oder mehrere ionische Bindungen verbunden sind.

Damit weisen die erfindungsgemäßen wasserunlöslichen Anionenaustauschermaterialien eine linear polymerisierte und/oder verzweigte und/oder vernetzte Struktur auf.

Dabei können die Anionenaustauschergruppen C untereinander und/oder mit Verbindungen sowohl nur linear polymerisiert sein und/oder verzweigt und/oder auch dreidimensional vernetzt, vorliegen. Dies bedeutet, dass, wenn nur Struktureinheiten gemäß den allgemeinen Formeln I bis IV vorliegen, diese nur linear chemisch und/oder verzweigt gekoppelt vorliegen können. Verzweigte Verbindungen der Anionenaustauschergruppen sollen linear polymerisierte Verbindungen mit Seitenketten sein, die untereinander im Wesentlichen nicht chemisch gekoppelt vorliegen.
Im Falle, dass Struktureinheiten gemäß den allgemeinen Formeln V bis VIII vorliegen, liegen zwar, in Abhängigkeit von der Länge der eingesetzten Struktureinheiten, auch lineare Teile in den Struktureinheiten vor, die auch verzweigt sein können, aber an den Vernetzungspunkten sind die Struktureinheiten gemäß den allgemeinen Formeln V bis VIII immer dreidimensional chemisch gekoppelt und damit vernetzt

Die Struktureinheiten gemäß den allgemeinen Formeln I bis VIII sind in jedem Fall mit wasserunlöslichen, vorteilhafterweise aromatischen oder aliphatischen und unter Einsatzbedingungen inerten, Polymeren B gekoppelt, wobei die Anionenaustauschergruppen C mindestens an einem Ende der Polymere B über die Verbindungsstelle X und/oder über K, wenn K gleich X ist, gekoppelt sind. Das andere Ende der Polymerkette bildet die Endgruppe Z oder K, die eine temperatur-und alkalistabile Verbindung, vorteilhafterweise eine niedermolekulare aromatische oder aliphatische, unter Einsatzbedingungen inerte Verbindung, ist.

Bei den Struktureinheiten gemäß den allgemeinen Formeln V bis VIII sind die Anionenaustauschergruppen C über die Vernetzungsstellen V miteinander dreidimensional chemisch gekoppelt, also vernetzt, wobei im Wesentlichen an den jeweiligen Enden der Polymerketten, auch an möglichen Seitengruppen, K als Endgruppen Z gekoppelt vorliegen, die eine temperaturstabile und alkalistabile Verbindung, vorteilhafterweise eine niedermolekulare aromatische oder aliphatische, unter Einsatzbedingungen inerte Verbindung ist.
Im Falle der Struktureinheiten gemäß den allgemeinen Formeln V bis VIII sind erfindungsgemäß mindestens ein K eine Verbindungsstelle X, die mit wasserunlöslichen, vorteilhafterweise aromatischen oder aliphatischen und unter Einsatzbedingungen inerten, Polymeren B gekoppelt sind, wobei gleichzeitig mindestens ein K eine Endgruppe Z darstellt, die nicht mit wasserunlöslichen, vorteilhafterweise aromatischen oder aliphatischen und unter Einsatzbedingungen inerten, Polymeren B gekoppelt vorliegt.

Vorteilhafterweise sind mehr K Verbindungsstellen X, die mit wasserunlöslichen, vorteilhafterweise aromatischen oder aliphatischen und unter Einsatzbedingungen inerten, Polymeren B gekoppelt sind, als nicht mit wasserunlöslichen, vorteilhafterweise aromatischen oder aliphatischen und unter Einsatzbedingungen inerten, Polymeren B gekoppelte K. Wenn K eine Endgruppe Z ist, ist diese jedoch nicht mit Polymer B gekoppelt.

Als Endgruppen Z können vorteilhafterweise Alkylthioether oder Arylthioether oder Benzylthioether vorhanden sein.

Die Verbindungsstelle X kann vorteilhafterweise N,N-Diallylpiperidiniumether oder N,N,Diallylpyrrolidiniumether oder N,N-Diallylaminoethylether oder Methacrylamidethylether oder Methacrylsäureester oder Methacrylsäurehydroxypropylether oder Xylylenether oder Phenylensulfonether sein.

Bei der Verbindungsstelle X sind eine, mehrere oder alle Verbindungsstellen X mit ihrem jeweiligen Partner über kovalente chemische Bindungen gekoppelt. Vorteilhafterweise ist die überwiegende Menge an Verbindungsstellen X mit ihren Partnern über kovalente chemische Bindungen gekoppelt.

In den erfindungsgemäßen Anionenaustauschermaterialien kann die Kopplung zwischen den Anionenaustauschergruppen C gemäß der allgemeinen Formeln I bis VIII und dem wasserunlöslichen Polymer B auch über ionische Bindungen zwischen den Anionenaustauschergruppen C und sauren Gruppen der Polymere B erfolgen.

Die Vernetzungsstelle V ist vorteilhafterweise Tetraallylammoniumchlorid oder Tetraalkylallylammoniumchlorid oder Diallyl-Dialkylallylammoniumchlorid oder 1,4-Divinylbenzol oder Divinylsulfon oder Divinylsulfid oder Divinylsulfoxid oder Divinylether oder Diacrylamid oder Dimethacrylamid oder N,N,N',N'-Tetraallyl-4,4'-Trimethylendipiperidiniumchlorid oder N,N,N',N'-Tetraalkylallyl-4,4'- Trimethylendipiperidininiumchlorid oder N,N-Diallyl-N',N'Dialkylallyl-4,4'- Trimethylendipiperidininiumchlorid oder N-Allyl-N,N',N'T rialkylallyl-4,4'- Trimethylendipiperidininiumchlorid oder N,N,N'-Triallyl-N'-Alkylallyl- Trimethylendipiperidiniumchlorid oder N,N,N',N'-Tetraallylpiperazininiumchlorid oder N,N,N',N'-Tetraalkylallylpiperazininiumchlorid oder N,N-Diallyl-N',N'Dialkylallylpiperazininiumchlorid oder N-Allyl-N,N',N'Trialkylallylpiperazininiumchlorid oder N,N,N'-Triallyl-N'-Alkylallylpiperazininiumchlorid. An Stelle der oben genannten Ammoniumchloride können auch deren Bromide oder Jodide eingesetzt werden.

Im Falle, dass D nichts und/oder m = 0 sind, bedeutet dies im Rahmen der vorliegenden Erfindung, dass das cyclische Strukturelement der Anionenaustauschergruppe C ein Fünfring ist.

Das wasserunlösliche Polymer B, welches vorteilhafterweise auch temperatur-und/oder alkalistabil ist, ist vorteilhafterweise Polyethersulfon oder Polysulfon oder Polyphenylen oder Polyphenylenether oder Polyphenylensulfid oder Poly(perfluorethylenpropylen) oder Polytetrafluorethylen Poly(ethylentetrafluorethylen) oder Perfluoralkoxypolymere oder Polystyrol oder Polyethylen oder Polypropylen.

Ebenfalls vorteilhafterweise ist das Comonomer A Styrol und/oder α-Methylstyrol und/oder N-Vinylcarbazol und/oder Methacrylester und/oder N-Vinylpyrrolidon und/oder N,N-Diallylacrylamid und/oder N,N-Diallylacrylsulfonamid und/oder Diallylether und/oder 1,2-Diallylbenzol und/oder Diallylsulfid und/oder Chlortrifluorethylen und/oder Tetrafluorethylen und/oder Hexafluorpropylen und/oder 1,2-Divinylbenzol ist.
Das Comonomer A ist dabei vorteilhafterweise zur räumlichen Trennung von ladungstragenden Gruppen vorhanden.

Vorteilhafterweise sind R₁ Stickstoff, R₆ bis R₉ Wasserstoff, R₂ und/oder R₃ Wasserstoff oder ein Methylrest, und R₄ und/oder R₅ ein Methyl- oder Ethylrest.

Die erfindungsgemäßen wasserunlöslichen Anionenaustauschermaterialien werden dadurch erreicht, dass die Stickstoff- oder Phosphorenthaltenden Anionenaustauschergruppen C der erfindungsgemäßen wasserunlöslichen Anionenaustauschermaterialien in die cyclische (heterocyclische) oder spirocyclische Verbindungen der Struktureinheiten gemäß mindestens einer der allgemeinen Formeln I bis VIII eingebunden sind.
Zusätzlich können β-ständige Wasserstoffatome zum positiv geladenen Atom der Anionenaustauschergruppen C in den Struktureinheiten gemäß mindestens einer der allgemeinen Formeln I bis VIII durch Alkyl- oder Arylreste substituiert sein.
Durch die Einbindung der Anionenaustauschergruppen C in die Struktureinheiten gemäß mindestens einer der allgemeinen Formeln I bis VIII als Segmente in linearen Blockcopolymeren, die auch durch polymerisierte Seitenketten eine bürstenartige (brushlike) Form aufweisen, oder durch Herstellung von dreidimensional vernetzten Polymeren daraus und dem Vorhandensein von Endgruppen Z (in den Formeln V bis VIII K = Z) und der Kopplung über Verbindungsstellen X (in den Formeln V bis VIII K = X) mit wasserunlöslichen Polymeren B liegen die erfindungsgemäßen wasserunlöslichen Anionenaustauschermaterialien vor.
Die verbesserte Alkali- und Temperaturstabilität der erfindungsgemäßen wasserunlöslichen Materialien ermöglicht die Erschließung neuer Anwendungsfelder oder eine verbesserte Effizienz bekannter Anwendungen.

Die erfindungsgemäßen wasserunlöslichen Anionenaustauschermaterialien können für Anionenaustauschermembranen oder sogenannten Anionenaustauscherharzen eingesetzt werden. Dabei bestehen die Anionenaustauschermembranen im Wesentlichen, aber mindestens überwiegend aus linearen Anionenaustauschergruppen C, die Bestandteil der Struktureinheiten gemäß mindestens einer der allgemeinen Formeln I bis IV sind. Anionenaustauscherharze bestehen im Wesentlichen, aber mindestens überwiegend aus vernetzten Anionenaustauschergruppen C, die Bestandteil der Struktureinheiten gemäß mindestens einer der allgemeinen Formeln V bis VIII sind.

Eine weitere vorteilhafte Eigenschaft der erfindungsgemäßen wasserunlöslichen Anionenaustauschermaterialien ist deren Geruchlosigkeit.

Die erfindungsgemäßen wasserunlöslichen Anionenaustauschermaterialien werden durch Cyclopolymerisation hergestellt.

Vorteilhafterweise werden als Monomere für die Herstellung der Anionenaustauschergruppen C N,N-Diallyl- und/oder N,N-Dialkylallylverbindungen und/oder N-Allyl-N-Alkylallyl-Verbindungen sekundärer aliphatischer oder aromatischer oder cycloaliphatischer Amine eingesetzt, wie beispielsweise Diallyldimethylammoniumchlorid, Diallylpiperidiniumchlorid, Diallylpyrrolidiniumchlorid, Allyl-Methallyldimethylammoniumchlorid, Allyl-Methallylpiperidiniumchlorid, Allyl-Methallylpyrrolidiniumchlorid, Dimethallyl-dimethylammoniumchlorid, Dimethallylpiperidiniumchlorid, Dimethallylpyrrolidiniumchlorid, Diallyl-3,4-Dimethylpyrrolidiniumchlorid, Diallyl-3,3,4,4-Tetramethylpyrrolidiniumchlorid, Diallyl-3,5-Dimethylpiperidiniumchlorid, Diallyl-3,3,5,5-Tetramethylpiperidiniumchlorid, Diallyl-Diphenylammoniumchlorid. Ebenfalls vorteilhafterweise können als solche Monomere P,P-Diallyl- und/oder P,P-Dialkylallylverbindungen und/oder P-Allyl-P-Alkylallyl-Verbindungen sekundärer aliphatischer oder aromatischer oder cycloaliphatischer Phosphine eingesetzt werden, wie beispielsweise Diallyldimethylphophiumchlorid, Diallyldiphenylphosphoniumchlorid. An Stelle der Ammoniumchloride oder Phosphoniumchloride können auch die entsprechenden Bromide oder Jodide eingesetzt werden.

Die erfindungsgemäßen wasserunlöslichen Anionenaustauschermaterialien weisen eine deutlich höhere Temperaturstabilität, insbesondere unter alkalischen Bedingungen auf, gegenüber Materialien von vergleichbaren Membranen nach dem Stand der Technik. Damit können die erfindungsgemäßen Anionenaustauschermaterialien in den bestehenden Anwendungsbereichen für solche Materialien problemlos eingesetzt werden und so zur Verbesserung der Effizienz dieser Verfahren führen. Durch die verbesserten Eigenschaften können sich auch neue Anwendungsgebiete für die erfindungsgemäßen Materialien eröffnen.

Nachfolgend wird die Erfindung an zwei Ausführungsbeispielen näher erläutert.

### Beispiel 1

### Herstellung einer Anionenaustauschermembran aus mehreren Anionenaustauschergruppen C der Struktureinheit gemäß der allgemeinen Formel II mit Kopplung über ein wasserunlösliches Polymer B

Zur Herstellung der Anionenaustauschermembran wird zuerst das wasserunlösliche Polymer B hergestellt. Dazu werden 52 mmol 4,4'-Difluordiphylsulfon und 50 mmol 4,4'-Bis-Trimethylsiloxydiphenylsulfon in 50 ml N-Methylpyrrolidon (NMP) gelöst und nachfolgend mit 75 mmol Kaliumcarbonat versetzt. Diese Mischung wird für 16 h bei 175°C unter Argonatmosphäre gerührt. Anschließend wird die Temperatur für 2 Stunden auf 190°C erhöht und weitere 100 mg 4,4'-Difluordiphenylsulfon zugegeben. Nach dem Abkühlen wird das Reaktionsprodukt in 1000 ml Ethanol unter Zusatz von 5 ml 37 %-iger Salzsäure gefällt. Dieses Produkt wird abgesaugt und mit 250 ml Ethanol für 5 h bei 50 °C gewaschen, abgesaugt und bei 100 °C im Vakuum getrocknet. Das Endprodukt ist ein Fluor-terminiertes Polyethersulfon- (Polymer B) mit einem Molekulargewicht Mₙ, ermittelt durch ¹H-NMR-Spektroskopie, von 11.000 g/mol und enthält keine OH-Endgruppen.

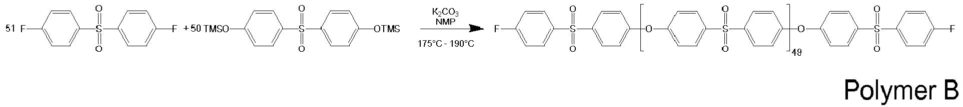

Zur Herstellung der Verbindungsstellen X an dem Polymer B gemäß der nachfolgend dargestellten Reaktion werden 2 mmol Polyethersulfon und 4 mmol 4-Hydroxypiperidin in 50 ml NMP gelöst und mit 6 mmol Kaliumcarbonat versetzt. Dieser Ansatz wird für 16 h bei 175°C gerührt und das Zwischenprodukt anschließend mit 500 ml Ethanol gefällt. Das dabei erhaltene Polymer wird für 5 h bei 50 °C in Ethanol gerührt, abfiltriert und im Vakuum bei 100 °C getrocknet.

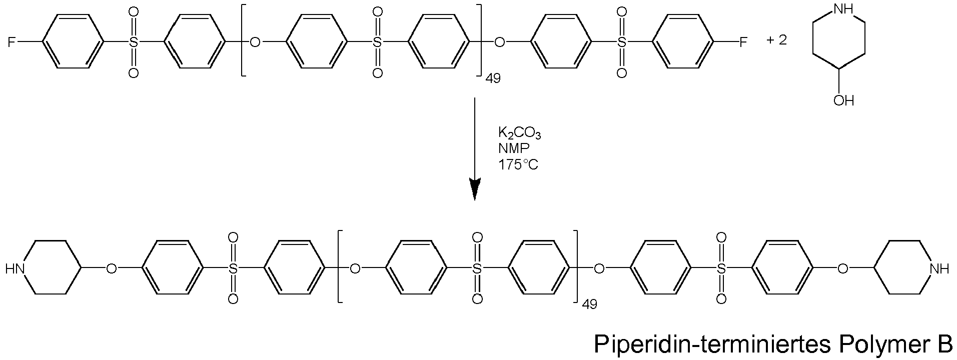

Das Endprodukt ist ein Piperidin-terminiertes Polyethersulfon. Die Ausbeute beträgt 90 %. Die vollständige Umsetzung der Fluor-Endgruppen mit 4-Hydroxypiperidin wurde durch ¹H-NMR-Spektroskopie nachgewiesen.
0,5 mmol dieses Piperidin-terminierten Polyethersulfons werden in 25 ml Dimethylsulfoxid (DMSO) gelöst und mit 4 mmol Kaliumcarbonat versetzt und auf 75 °C erhitzt. Über einen Zeitraum von 30 min werden 4 mmol Allylchlorid, gelöst in 10 ml DMSO, zu der Reaktionsmischung getropft. Nachdem das in DMSO gelöste Allylchlorid vollständig zugegeben wurde, wird die Mischung für 24 h bei 90 °C gerührt. Nach dem Abkühlen wird das mit der Verbindungsstelle X Diallylpiperidinium-terminierte Polyethersulfon in Ethanol gefällt, in frischem Ethanol bei 50 °C gerührt und abschließend abfiltriert. Das Produkt wird im Vakuum bei 50 °C getrocknet. Die Ausbeute beträgt 85 %. Die vollständige Bildung der terminalen quaternären Ammoniumsalze wurde durch ¹H-NMR-Spektroskopie ermittelt.

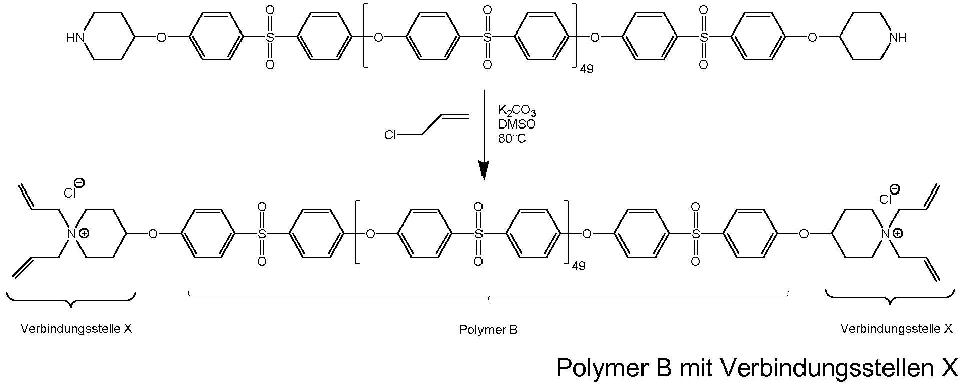

Danach werden zur Herstellung der Verbindung mit mehreren Anionenaustauschergruppen C, gemäß der Struktureinheit nach der allgemeinen Formel II, 0,1 mmol des Diallylpiperidinium-terminierten Polyethersulfons (Polymer B mit Verknüpfungsstellen X) und 4 mmol N,N-Diallylpyrrolidiniumchlorid (Anionenaustauschergruppe C gemäß Formel II, hergestellt analog der Vorschrift nach de Vynck et al. Macromol. Rapid Commun. 18, 1997, 149-156) in 50 ml DMSO gelöst und durch vier Gefrier-Auftauzyklen von gelöstem Sauerstoff befreit. Die Reaktionslösung wird auf 90 °C erwärmt und mit 4 mol-% AIBN versetzt, 24 h bei 90 °C unter Argonatmosphäre gerührt. Nach dem Abkühlen wird das entstandene Polyethersulfon-Poly(azoniaspiro[4,4]nonan-Blockpolymer zur Absättigung der endständigen Doppelbindungen mit Thiophenol in Gegenwart von Kupfer(I)salz behandelt und anschließend in Wasser gefällt. Bei der Reaktion möglicherweise entstandenes Homopolymer wird durch Extraktion mit Wasser in einer Soxhlett-Apparatur entfernt. Die Zusammensetzung, die mittels ¹H-NMR-Spektroskopie ermittelt wurde, entspricht der Monomerzusammensetzung gemäß der Formel II. Die Ausbeute beträgt 95 %.

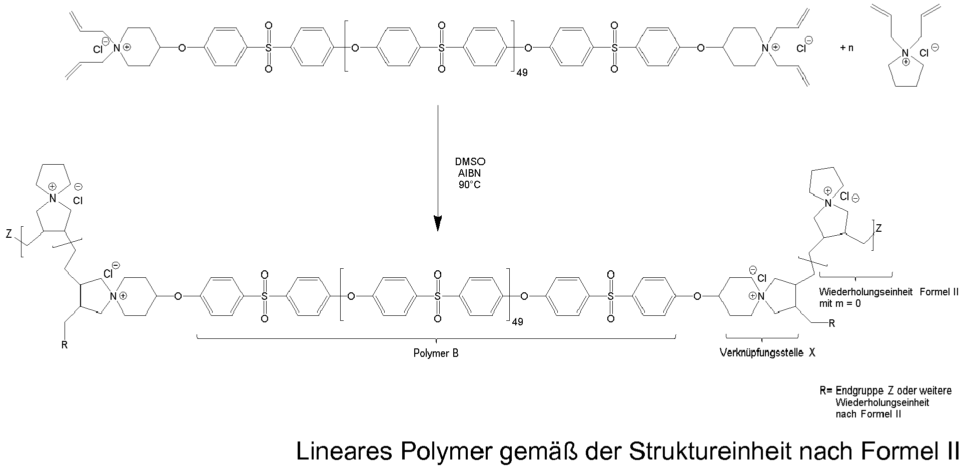

Anschließend wird aus dem so hergestellten Polyethersulfon-Poly(azoniaspiro[4,4]nonan-Blockpolymer eine Membran hergestellt. Dazu werden 0,5 g Polyethersulfon-Poly(azoniaspiro[4,4]nonan-Blockpolymer in 10 ml N-Methylpyrrolidon gelöst, filtriert und in eine Petrischale mit 7 cm Durchmesser gegeben. Das Lösungsmittel N-Methylpyrrolidon wird im Vakuum für 2 h bei Raumtemperatur, anschließend für 2 h bei 40 °C, weitere 2 h bei 60 °C und dann für 24 h bei 100°C entfernt. Die resultierende Membran wird mit Wasser aus der Petrischale gelöst, in Wasser bei 70°C für 4 h gewaschen und abschließend im Vakuum bei 50 °C zur Gewichtskonstanz getrocknet.

Die Membran aus Polyethersulfon-Poly(azoniaspiro[4,4]nonan)-Blockcopolymer weist eine Dicke (trocken) = 70 µm, eine lonenaustauscherkapazität = 1,30 mmol/g, eine Wasseraufnahme bei 80 °C = 65 % und eine Hydroxidionenleitfähigkeit (80 °C, 95 % rel. Feuchte) = 70 mS/cm auf.

Das Membranmaterial Polyethersulfon-Poly(anoniaspiro[4,4]nonan)-Blockcopolymer wird bezüglich seiner Temperaturstabilität charakterisiert. Dazu werden 0,5 g der Membran aus Polyethersulfon-Poly(anoniaspiro[4,4]nonan)-Blockcopolymer zunächst für 24 Stunden in 2M Natronlauge gelagert, um das Anionenaustauschermaterial in die OH-Form zu überführen. Anschließend wird die Membran in 5 ml 2N Natronlauge gegeben, das Gefäß abgeschmolzen und der Inhalt 168 h bei einer Temperatur von 120°C gehalten. Nach dem Abkühlen wird das Gefäß geöffnet und das Anionenaustauschermaterial mit Wasser gewaschen und anschließend 24 h in 1M Natriumchloridlösung gelagert. Die Probe wird nochmals mit Wasser gewaschen und im Vakuum bei 50 °C bis zur Gewichtskonstanz getrocknet.

Das Membranmaterial zeigte dabei folgende Ergebnisse, wobei die Werte jeweils vor und nach der Bestimmung der Temperaturstabilität ermittelt wurden:
Masse
   vor Test = 0,5 g, nach Test = 0,49 g,
   lonenaustauscherkapazität
   vor Test = 1,30 mmol/g, nach Test = 1,29 mmol/g,
Hydroxidionenleitfähigkeit (80 °C, 95 % rel. Feuchte)
   vor Test = 70 mS/cm, nach Test = 69 mS/cm.

Die Unterschiede der Ergebnisse liegen im Fehlerbereich des jeweiligen Messverfahrens

### Beispiel 2

### Herstellung einer Anionenaustauschermembran aus einer vernetzten Verbindung mit mehreren Anionenaustauschergruppen C, die Bestandteil der Struktureinheiten gemäß der allgemeinen Formel VI sind mit Kopplung über ein wasserunlösliches Polymer B

Zur Herstellung der Anionenaustauschermembran wird zuerst das wasserunlösliche Polymer B mit K, die eine Verbindungsstelle X darstellt, gemäß Beispiel 1 hergestellt. Es wird eine Lösung von 0,1 mmol Polymer B, 10 mmol N,N-Diallylpiperidiniumchlorid und 2 mmol N,N,N,N-Tetraalyllammoniumchlorid als Vernetzungsstelle V und 0,1 mmol 2,4,6-Trimethylbenzoyldiphenylphosphinoxid (DPO) als UV-Initiator, in 10 mL Dimethylsulfoxid gelöst und sorgfältig entgast. Diese Lösung wird in eine Petrischale mit 7 cm Durchmesser gegeben, die mit einer Quarzglasscheibe abgedeckt wird, und für 10 min mit UV-Licht mit einer Wellenlänge von 254 nm (Leistung 8 W) bei Raumtemperatur bestrahlt. Anschließend wird das Lösungsmittel im Vakuum bei einer Temperatur von 100 bis 120 °C entfernt. Das Anionenaustauschermaterial wird mit Wasser gewaschen und zur Absättigung der endständigen Doppelbindungen mit Thiophenol in Gegenwart von Kupfer(I)salz behandelt. Die Anionenaustauschermembran wird nochmals gründlich mit Wasser gewaschen und im Vakuum bis zur Gewichtskonstanz getrocknet. Die Membran weist eine Dicke von 100 µm (trocken), eine lonenaustauscherkapazität von 3,35 mmol/g, eine Wasseraufnahme bei 80°C von 100% und eine Hydroxydionenleitfähigkeit (80°C, 95% rel. Feuchte) von 90 mS/cm auf.

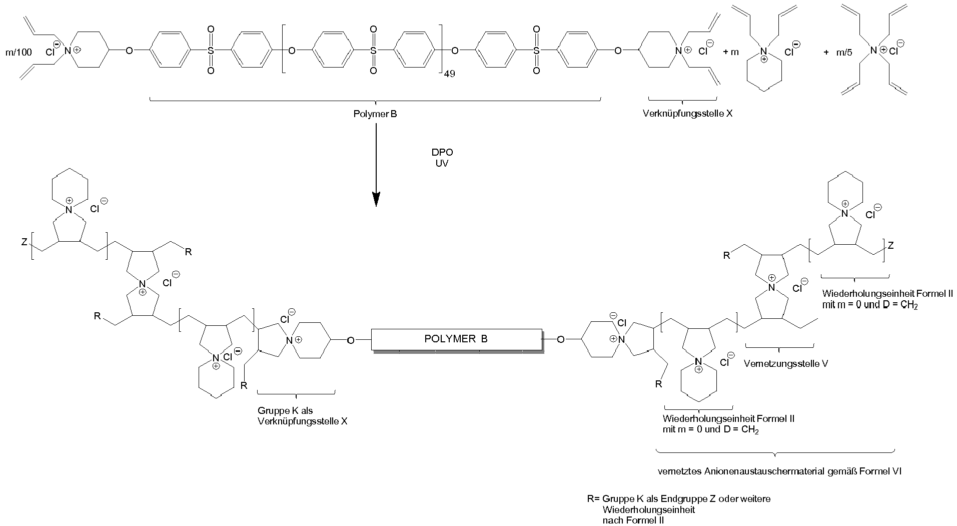

Das Membranmaterial wird bezüglich seiner Temperaturstabilität charakterisiert. Dazu werden 0,5 g der Membran zunächst für 24 Stunden in 2M Natronlauge gelagert, um das Anionenaustauschermaterial in die OH-Form zu überführen. Anschließend wird die Membran in 5 ml 2N Natronlauge gegeben, das Gefäß abgeschmolzen und der Inhalt 168 h bei einer Temperatur von 120°C gehalten. Nach dem Abkühlen wird das Gefäß geöffnet und das Anionenaustauschermaterial mit Wasser gewaschen und anschließend 24 h in 1 M Natriumchloridlösung gelagert. Die Probe wird nochmals mit Wasser gewaschen und im Vakuum bei 50 °C bis zur Gewichtskonstanz getrocknet.

Das Membranmaterial zeigte dabei folgende Ergebnisse, wobei die Werte jeweils vor und nach der Bestimmung der Temperaturstabilität ermittelt wurden:
Masse
   vor Test = 0,5 g, nach Test = 0,49 g,
lonenaustauscherkapazität
   vor Test = 3,35 mmol/g, nach Test = 3,32 mmol/g,
Hydroxidionenleitfähigkeit (80 °C, 95 % rel. Feuchte)
   vor Test = 90 mS/cm, nach Test = 88 mS/cm.
Die Unterschiede der Ergebnisse liegen im Fehlerbereich des jeweiligen Messverfahrens

## Patentansprüche

1. Wasserunlösliche Anionenaustauschermaterialien, mindestens bestehend aus miteinander linear polymerisierten und/oder verzweigten und/oder vernetzten Anionenaustauschergruppen C, die Bestandteil der Struktureinheiten gemäß mindestens einer der allgemeinen Formeln I bis VIII 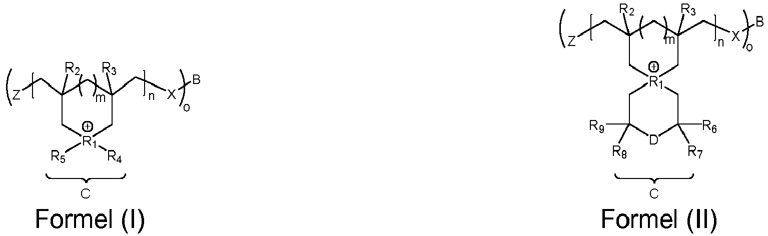 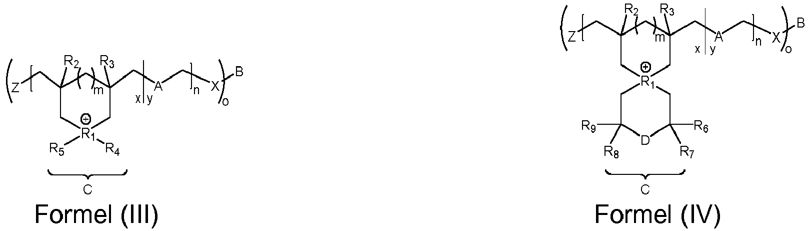 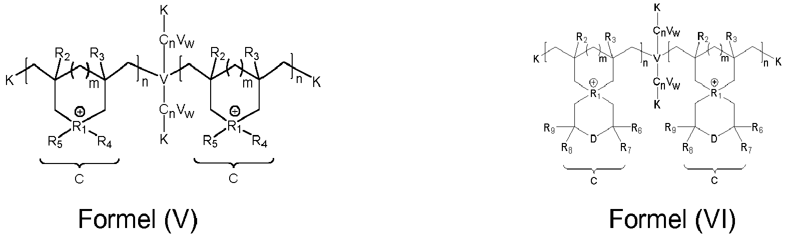 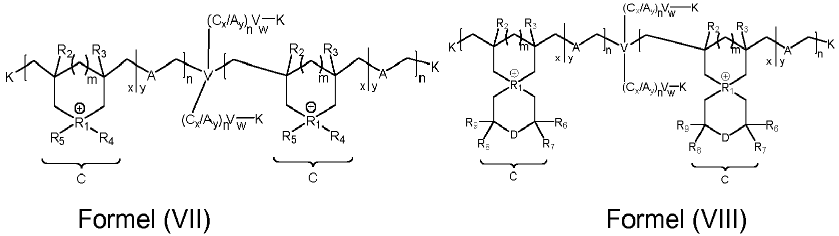 sind, mit
- R₁ Stickstoff und/oder Phosphor
- R₂ - R₉ Substituenten, wobei
∘ R₂ und R₃ Wasserstoff sind oder, im Falle dass mindestens ein Rest R₂ und R₃ ungleich Wasserstoff ist, dann R₂ und/oder R₃ ein Alkylrest oder Arylrest ist, und,
∘ R₄ und R₅ jeweils ein Alkyl- und/oder Arylrest ist, und
∘ R₆ - R₉ Wasserstoff sind, oder im Falle dass mindestens ein Rest R₆ bis R₉ ungleich Wasserstoff ist, dann diese Reste R₆ bis R₉ ein Alkylrest oder Arylrest ist, und
- Z eine Endgruppe ist, und
- X eine Verbindungsstelle ist, und
- V eine Vernetzungsstelle ist, und
- K eine Endgruppe Z oder eine Verbindungsstelle X ist, und
- D nichts ist oder mindestens eine Methylengruppe oder Sauerstoff oder Schwefel ist, und
- B ein wasserunlösliches temperaturstabiles und alkalistabiles Polymer ist, und
- A ein Comonomer ist, das keine funktionelle Gruppe aufweist, und
- 2 ≤n ≤ 100 ist, und
- m = 0 oder 1 ist, und
- 1 ≤ o ≤ 100 ist, und
- 50 mol% ≤ x ≤ 99 mol% und y = (100 mol% -x) ist, und
- 10 mol% ≤ w ≤ 100 mol% und (n + w) = 100mol% ist,
wobei
- im Falle von K mindestens eine Endgruppe Z und mindestens eine Verbindungsstelle X vorhanden sind, und
- die Anionenaustauschergruppen C als Bestandteil der Struktureinheiten gemäß den allgemeinen Formeln I bis IV über eine, mehrere oder alle Verbindungsstellen X an ein wasserunlösliches Polymer B gekoppelt sind und im Wesentlichen alle Endgruppen Z eine temperatur- und alkalistabile Verbindung sind,
und/oder
- die Anionenaustauschergruppe C als Bestandteil der Struktureinheiten gemäß den allgemeinen Formeln V bis VIII über die Vernetzungsstelle V miteinander vernetzt sind, und im Wesentlichen alle Endgruppen Z eine temperatur- und alkalistabile Verbindung sind und alle K, die eine Verbindungsstelle X sind, mit einem wasserunlöslichen Polymer B über kovalente chemische Bindungen gekoppelt sind,
und/oder
- die Anionenaustauschergruppe C und das wasserunlöslichen Polymer B über eine oder mehrere ionische Bindungen verbunden sind.

2. Wasserunlösliche Anionenaustauschermaterialien nach Anspruch 1, bei denen mindestens zwei Anionenaustauschergruppen C gemäß der Formel I-IV mit einem wasserunlöslichen Polymer B über eine Verbindungsstelle X über kovalente chemische Bindungen miteinander gekoppelt sind und/oder die Anionenaustauschergruppen C über ionische Bindungen mit dem wasserunlöslichen Polymer B verbunden sind und die Endgruppen Z temperaturstabile und alkalistabile Verbindungen sind.

3. Wasserunlösliche Anionenaustauschermaterialien nach Anspruch 1, bei denen die Verbindungsstelle X N,N-Diallylpiperidiniumether oder N,N-Diallylpyrrolidiniumether oder N,N-Diallylaminoethylether oder Methacrylamidethylether oder Methacrylsäureester oder Methacrylsäurehydroxypropylether oder Xylylenether oder Phenylenethersulfon ist.

4. Wasserunlösliche Anionenaustauschermaterialien nach Anspruch 1, bei denen Anionaustauschergruppen C gemäß der Formel V-VIII über Vernetzungsstellen V vernetzt sind und K eine Verbindungsstelle X ist, an die das wasserunlösliche Polymer B über kovalente chemische Bindungen gekoppelt ist.

5. Wasserunlösliche Anionenaustauschermaterialien nach Anspruch 1, bei denen die Endgruppen Z Alkylthioether oder Arylthioether oder Benzylthioether sind.

6. Wasserunlösliche Anionenaustauschermaterialien nach Anspruch 1, bei denen das wasserunlösliche, temperaturstabile und alkalistabile Polymer B Polyethersulfon oder Polysulfon oder Polythioethersulfon oder Polyphenylen oder Polyphenylenether oder Polyphenylensulfid oder Poly(perfluorethylenpropylen) oder Polytetrafluorethylen Poly(ethylen-tetrafluorethylen) oder Perfluoralkoxypolymere oder Polystyrol oder Polyethylen oder Polypropylen oder ein sulfoniertes und/oder carboxyliertes und/oder phosphoniertes Polymer des Typs Polyethersulfon oder Polysulfon oder Polyphenylen oder Polyphenylenether oder Polyphenylensulfid oder Poly(perfluorethylenpropylen) oder Polytetrafluorethylen Poly(ethylentetrafluorethylen) oder Perfluoralkoxypolymere oder Polystyrol oder Polyethylen oder Polypropylen ist.

7. Wasserunlösliche Anionenaustauschermaterialien nach Anspruch 1, bei denen das Comonomer A Styrol und/oder α-Methylstyrol und/oder N-Vinylcarbazol und/oder Methacrylester und/oder N-Vinylpyrrolidon und/oder N,N-Diallylacrylamid und/oder N,N-Diallylacrylsulfonamid und/oder Diallylether und/oder 1,2-Diallylbenzol und/oder Diallylsulfid und/oder Chlortrifluorethylen und/oder Tetrafluorethylen und/oder Hexafluorpropylen und/oder 1,2-Divinylbenzol ist.

8. Wasserunlösliche Anionenaustauschermaterialien nach Anspruch 1, bei denen die Vernetzungsstelle V Tetraallylammoniumchlorid oder Tetraalkylallylammoniumchlorid oder Diallyl-Dialkylallylammoniumchlorid oder 1,4-Divinylbenzol oder Divinylsulfon oder Divinylsulfid oder Divinylsulfoxid oder Divinylether oder Diacrylamid oder Dimethacrylamid oder N,N,N',N'-Tetraallyl-4,4'-Trimethylendipiperidininiumchlorid oder N,N,N',N'-Tetraalkylallyl-4,4'-Trimethylendipiperidiniumchlorid oder N,N-Diallyl-,N',N'Dialkylallyl-4,4'-Trimethylendipiperidiniumchlorid oder N-Allyl-N,N',N'Trialkylallyl-4,4'-Trimethylendipiperidiniumchlorid oder N,N,N'-Triallyl-N'-Alkylallyl-4,4'-Trimethylendipiperidiniumchlorid oder N,N,N',N'-Tetraallylpiperaziniumchlorid oder N,N,N',N'-Tetraalkylallylpiperaziniumchlorid oder N,N-Diallyl-N',N'Dialkylallylpiperaziniumchlorid oder N-Allyl-N,N',N'Trialkylallylpiperaziniumchlorid oder N,N,N'-Triallyl-N'-Alkylallylpiperaziniumchlorid und/oder ein Bromid und/oder ein Jodid dieser Verbindungen ist.

9. Wasserunlösliche Anionenaustauschermaterialien nach Anspruch 1, bei denen als Monomere für die Herstellung der Anionenaustauschergruppen C N,N-Diallyl-und/oder N,N-Dialkylallylverbindungen und/oder N-Allyl-N-Alkylallyl-Verbindungen sekundärer aliphatischer oder aromatischer oder cycloaliphatischer Amine, wie beispielsweise Diallyldimethylammoniumchlorid, Diallylpiperidiniumchlorid, Diallylpyrrolidiniumchlorid, Allyl-Methallyldimethylammoniumchlorid, Allyl-Methallylpiperidiniumchlorid, Allyl-Methallylpyrrolidiniumchlorid, Dimethallyldimethylammoniumchlorid, Dimethallylpiperidiniumchlorid, Dimethyallylpyrrolidiniumchlorid, Diallyl-3,4-Dimethylpyrrolidiniumchlorid, Diallyl-3,3,4,4-Tetramethylpyrrolidiniumchlorid, Diallyl-3,5-Dimethylpiperidiniumchlorid, Diallyl-3,3,5,5-Tetramethylpiperidiniumchlorid, Diallyl-Diphenylammoniumchlorid P,P-Diallyl- und/oder P,P-Dialkylallylverbindungen und/oder P-Allyl-P-Alkylallyl-Verbindungen sekundärer aliphatischer oder aromatischer oder cycloaliphatischer Phosphine, wie beispielsweise Diallyldimethylphophiumchlorid, Diallyldiphenylphosphoniumchlorid und/oder ein Bromid und/oder ein Jodid dieser Verbindungen eingesetzt sind.

10. Wasserunlösliche Anionenaustauschermaterialien nach Anspruch 1, bei denen R₁ Stickstoff ist.

11. Wasserunlösliche Anionenaustauschermaterialien nach Anspruch 1, bei denen R₂ und/oder R₃ ein Methylrest und/oder Wasserstoff ist.

12. Wasserunlösliche Anionenaustauschermaterialien nach Anspruch 1, bei denen R₄ und R₅ ein Alkylrest ist und vorteilhaft ein Methyl- oder Ethylrest ist.

13. Wasserunlösliche Anionenaustauschermaterialien nach Anspruch 1, bei denen R₆ - R₉ Wasserstoff sind.

14. Wasserunlösliche Anionenaustauschermaterialien nach Anspruch 1, bei denen 10 ≤ n ≤ 50 ist.

15. Wasserunlösliche Anionenaustauschermaterialien nach Anspruch 1, bei denen 2 ≤ o ≤ 10 ist.
